(19) Europäisches Patentamt
European Patent Office
Office européen des brevets

(11) Veröffentlichungsnummer: **0 153 992 B1**

# (12) EUROPÄISCHE PATENTSCHRIFT

(45) Veröffentlichungstag der Patentschrift: **16.10.91**

(51) Int. Cl.[5]: **C07C 237/46**, A61K 49/04

(21) Anmeldenummer: **84112723.6**

(22) Anmeldetag: **22.10.84**

Die Akte enthält technische Angaben, die nach dem Eingang der Anmeldung eingereicht wurden und die nicht in dieser Patentschrift enthalten sind.

## (54) Wasserlösliche 3,5-Diacetamido-2,4,6-triiodbenzoesäurederivate sowie Verfahren zu deren Herstellung und Verwendung.

(30) Priorität: **29.02.84 DE 3407473**

(43) Veröffentlichungstag der Anmeldung:
**11.09.85 Patentblatt 85/37**

(45) Bekanntmachung des Hinweises auf die Patenterteilung:
**16.10.91 Patentblatt 91/42**

(84) Benannte Vertragsstaaten:
**AT BE CH FR GB IT LI LU NL SE**

(56) Entgegenhaltungen:
**DE-A- 2 031 724**
**DE-B- 2 261 584**
**US-A- 4 307 072**

(73) Patentinhaber: **Dr. Franz Köhler Chemie GmbH**
**Neue Bergstrasse 3 - 7**
**W-6146 Alsbach-Hähnlein 1(DE)**

(72) Erfinder: **Schuster, Dan-Karl, Dr.**
**Am Brunengarten 25/7**
**W-6800 Mannheim 1(DE)**
Erfinder: **Köhler, Franz, Dr.**

**verstorben(DE)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

**EP 0 153 992 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue, nichtionische Derivate von 3,5-Diacetamido-2,4,6-triiodbenzoesäure, die zum Teil durch hohe Löslichkeit in Wasser, durch Elektroneutralität ihrer wäßrigen Lösungen, durch verminderten osmotischen Druck im Vergleich mit den korrespondierenden Salzen der 3,5-Diacetamido-2,4,6-triiodbenzoesäure, durch praktikable Viskositäten und durch irrelevante Toxizitätszahlen charakterisiert sind.

Die neuen Verbindungen entsprechen der allgemeinen Formel

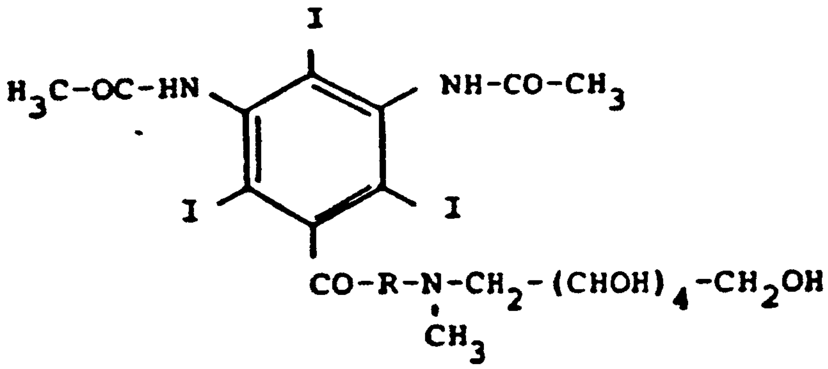

(I)

worin R einen Alanin-, Sarkosin-, Leucin-, Serin-, N-Lactyl-lysin, Asparaginsäure- oder Glutaminsäurerest bedeutet.

Bevorzugt sind weiterhin folgende Verbindungen:

N,N-[(3,5-Diacetamido-2,4,6-triiodbenzoyl)sarkosin]-N-methylglucamid (II);
N,N-[(3,5-Diacetamido-2,4,6-triiodbenzoyl)-L-alanin]-N-methylglucamid (III);
N,N-[(3,5-Diacetamido-2,4,6-triiodbenzoyl)-L-leucin]-N-methylglucamid (IV):
N,N-[(3,5-Diacetamido-2,4,6-triiodbenzoyl)-L-serin]-N-methylglucamid (V):
N,N-[(3,5-Diacetamido-2,4,6-triiodbenzoyl)-L-asparaginsäure]-bis-N-methylglucamid (VI);
N,N-[(3,5-Diacetamido-2,4,6-triiodbenzoyl)-DL-asparaginsäure]-bis-N-methylglucamid (VII);
N,N-[(3,5-Diacetamido-2,4,6-triiodbenzoyl)-L-glutaminsäure]-bis-N-methylglucamid (VIII);
N,N-[α-Lactyl-ε-(3,5-diacetamido-2,4,6-triiodbenzoyl)-lysin]-N-methylglucamid (IX);
N,N-[ε-Lactyl-α-(3,5-diacetamido-2,4,6-triiodbenzoyl)-lysin]-N-methylglucamid (X);
N-(3,5-Diacetamido-2,4,6-triiodbenzoyl)-aminosäuren der allgemeinen Formel XI und -aminosäurenester der allgemeine Formel XII, worin R den oben genannten Aminosäurerest, R' ein niedriger Alkyl- oder o- oder p-Nitrophenylester ist;
N-(3,5-Diacetamido-2,4,6-triiodbenzoyl)sarkosin (XIII), -sarkosinethylester (XIV), -sarkosin-o-nitrophenylester (XV);
N-(3,5-Diacetamido-2,4,6-triiodbenzoyl)-L-alanin (XVI), -L-alaninethylester (XVII), -L-alanin-o-nitrophenylester (XVIII);
N-(3,5-Diacetamido-2,4,6-triiodbenzoyl)-L-leucin (XIX), -L-leucinethylester (XX), -L-leucin-o-nitrophenylester (XXI);
N-(3,5-Diacetamido-2,4,6-triiodbenzoyl)-L-serin (XXII), -L-serinethylester (XXIII), -L-serin-o-nitrophenylester (XXIV);
N-(3,5-Diacetamido-2,4,6-triiodbenzoyl)-L-glutaminsäure (XXV), -L-glutaminsäurediethylester (XXVI);
N-[α-(O-Acetyl-lactyl)-ε-(3,5-diacetamido-2,4,6-triiodbenzoyl)]-L-lysinethylester (XXVII);
N-[ε-(O-Acetyl-lactyl-α-(3,5-diacetamido-2,4,6-triiodbenzoyl)]-L-lysinethylester (XXVIII);
N-[ε-Benzyloxycarbonyl-α-(3,5-diacetamido-2,4,6-triiodbenzoyl)]-L-lysinethylester (XXIX);
N-[ε-Benzyloxycarbonyl-α-(O-acetyl-lactyl)]-L-lysinethylester (XXX) mit der Formel

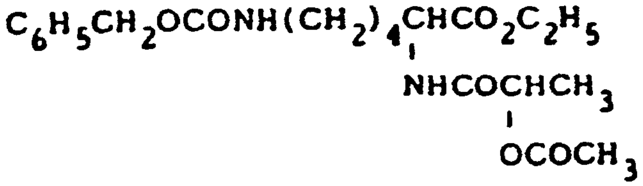
(XXX)

Besonders bevorzugt sind die Verbindungen (II), (VI), (VII) und (VIII).
Aus der US-A-4 307 072 sind nichtionische Röntgenkontrastmittel der Formel

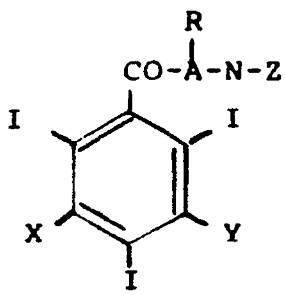

bekannt, worin X und Y nichtionische Funktionen darstellen, die mit niedriger Toxizität und/oder Wasserlöslichkeit in der 2,4,6-Triiodfunktion vereinbar sind. A ist ein mit Amino substituierter niederer Alkansäurekuppler und

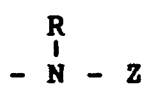

ist ein einwertiger Rest eines Polyhydroxyamins, worin N ein Stickstoffatom ist, R Wasserstoff, niederes Alkyl, Hydroxy-niederes Alkyl oder Polyhydroxy-niederes Alkyl, Z ein Polyhydroxy-niederes Alkyl oder Oxo, Polyhydroxy-niederes Alkyl.

Von H. Suter und H. Zutter sind in "Helv. Chim. Acta", 54 (1971), S. 2551-2559, bereits entsprechende Derivate von Glycinester, Methylaminoessigsäureester und Prolinester beschrieben. Diese Derivate sind jedoch einmal komplizieretei herzustellen als die erfindungsgemäßen, zum anderen entsprechen sie nicht den angegebenen Strukturen und sind ionisiert. Sie gehören somit zu einer vollständig anderen Klasse von Röntgenkontrastmitteln.

Die Konfiguration der α-Aminosäurekomponente kann also beliebig sein. Die Art des Aminosäurerestes beeinflußt in hohem Maße die Löslichkeit in Wasser:
die Verbindungen II, VI, VII und VIII sind sehr gut (über 100 %),
die Verbindungen III, IV und V sind gut (50 bis 80 %)
löslich. Die Wasserlöslichkeit ist also nicht nur abhängig von der Zahl der Hydroxy-Gruppen, sondern auch von der Art der N-Alkylierung der Aminosäurekomponente.
Das soll nicht heißen, daß die weniger löslichen Verbindungen in diagnostischer Hinsicht wertlos sind. In Bezug auf die Löslichkeit dominieren in der Tat die Verbindungen II, VI, VII und VIII. Die primären Forderungen an ein geeignetes Kotnrastmittel betreffen

a) optimale allgemeine und lokale Verträglichkeit,
b) geringe Toxizität,
c) minimale Viskosität,
d) ausreichende Löslichkeit,
e) geringen osmotischen Druck.

Diese Eigenschaften werden von nichtionischen Kontrastmitteln besser erreicht als bei den ionisierten Kontrastmitteln. Es muß also auf die ausgezeichnete Löslichkeit in Wasser, auf die relativ sehr guten

Viskositätsdaten, sowie auf die sehr geringe Toxizität hingewiesen werden. Der osmotische Druck ist bei nichtionisierten Kontrastmitteln ihrer Natur gemäß eo ipso geringer als bei vergleichbaren ionischen Formeln.

Die Derivate, die eine sehr gute bis gute Löslichkeit in Wasser besitzen, zeigen Viskositätswerte, die erwartungsgemäß von der Zahl und der Stellung der Hydroxy-Gruppen, sowie von der Konfiguration des Aminosäurerestes abhängig sind.

**Tabelle 1**

| Konzentration (mg J/ml) | Viskosität (cP) 20° C | | | 37°C | | |
|---|---|---|---|---|---|---|
| | Verbindungen | | | | | |
| | II | VI | VII | II | VI | VII |
| 100 | 2,16 | 2,90 | - | 1,48 | 1,84 | - |
| 150 | 3,50 | 5,15 | - | 2,22 | 3,04 | - |
| 200 | 5,74 | 11,14 | 10,65 | 3,32 | 6,06 | 5,84 |
| 250 | 10,84 | 33,58 | 25,38 | 6,20 | 15,96 | 12,23 |
| 300 | 18,88 | 88,85 | - | 9,22 | 35,41 | - |

**Tab. 1: Viskositätsdaten in Abhängigkeit von der Art der Aminosäure und der Konzentration des Präparates.**

Die wäßrigen Lösungen der erfindungsgemäß beschriebenen neuen nichtionischen Derivate der 3,5-Diacetamido-2,4,6-triiodbenzoesäure sind thermisch weitgehend stabil, so daß eine Hitzesterilisierung die Qualität nicht beeinträchtigt.

**Tabelle 2**

| Zeit min | Temp. °C | pH | Jodid mg J/100 ml | Farbe |
|---|---|---|---|---|
| Ausgangslösung | | 7,85 | 1,78 | gelblich |
| 30 | 110 | 7,85 | 2,63 | gelblich |
| 60 | 110 | 7,75 | 3,72 | gelblich |
| 30 | 120 | 7,80 | 3,73 | gelblich |

**Tab. 2: Verhalten der 67,9 % wäßrigen Lösung von II bei unterschiedlichen Temperaturen und Zeiten.**

Im Verhältnis zu den vergleichbaren ionisierten Na-, N-Methylglucamin- oder Lysin-Salzen der 3,5-Diacetamido-2,4,6-triiodbenzoesäure zeichnen sich die erfindungsgemäßen Derivate durch eine Halbierung des osmotischen Drucks aus, wodurch die allgemeine und lokale Verträglichkeit entscheidend verbessert ist.

Auch die Toxizitätsdaten liegen erheblich über denen der ionischen Verbindungen, wie Tabelle 3 zu entnehmen ist.

**Tabelle 3**

| Substanz | $LD_{50}$ (g/kg) |
|---|---|
| Natrium-Meglumin-Diatrizoat | 12 - 14 |
| Lysin-diatrizoat | 14 - 18 |
| Diatrizoyl-sarkosin-N-methylglucamid (II) | 36 |

**Tab. 3: $LD_{50}$ bei Maus, i.v. (14 Tage) nach Applikation der Lösung mit einem Gehalt von 300 mg J/ml.**

Das Verfahren zur Herstellung der neuen Derivate gemäß der allgemeinen Formel I ist dadurch gekennzeichnet, daß man einen Ester der allgemeinen Formel

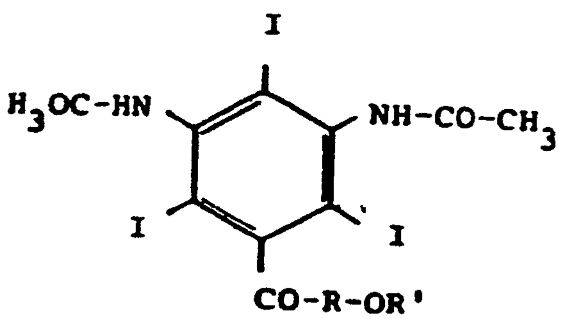

(XII)

worin R einen Alanin-, Sarkosin-, Leucin-, Serin-, N-Lactyl-lysin-, Asparaginsäure-, Glutaminsäurerest bedeutet, R' ein Alkyl- oder o- oder p-Nitrophenylesterrest ist, in an sich bekannter Weise mit N-Methylglucamin in einem kationsolvatisierenden Lösungmittel, wie N,N-Dimethylformamid, N,N-Dimethylacetamid oder Dimethylsulfoxid in Gegenwart eines Alkalicarbonats, beispielsweise bei Temperaturen von 20 bis 80 °C, reagieren läßt.

Alle Derivate, mit Ausnahme der zwei Lysinderivate, wurden nach dem nachstehenden Schema synthetisiert:

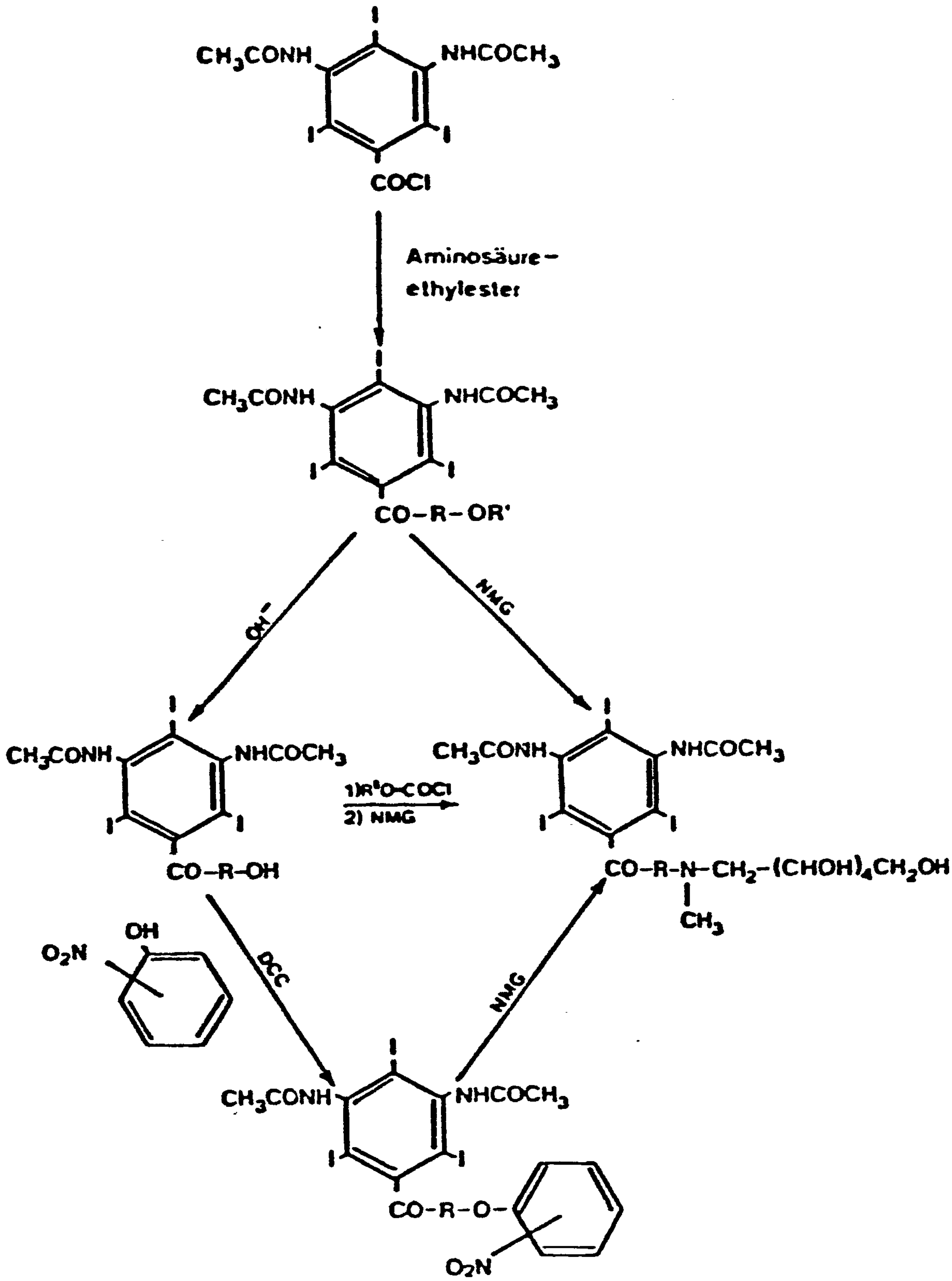


worin

| | |
|---|---|
| R = | Aminosäurerest |
| R' = | $C_2H_5$ |
| R'' = | $C_2N_5$, i = $C_4H_9$ |
| DCC = | N,N-Dicyclohexylcarbodiimid |
| NMG = | N-Methylglucamin |

Die zwei Lysinderivate wurden folgendermaßen synthetisiert:

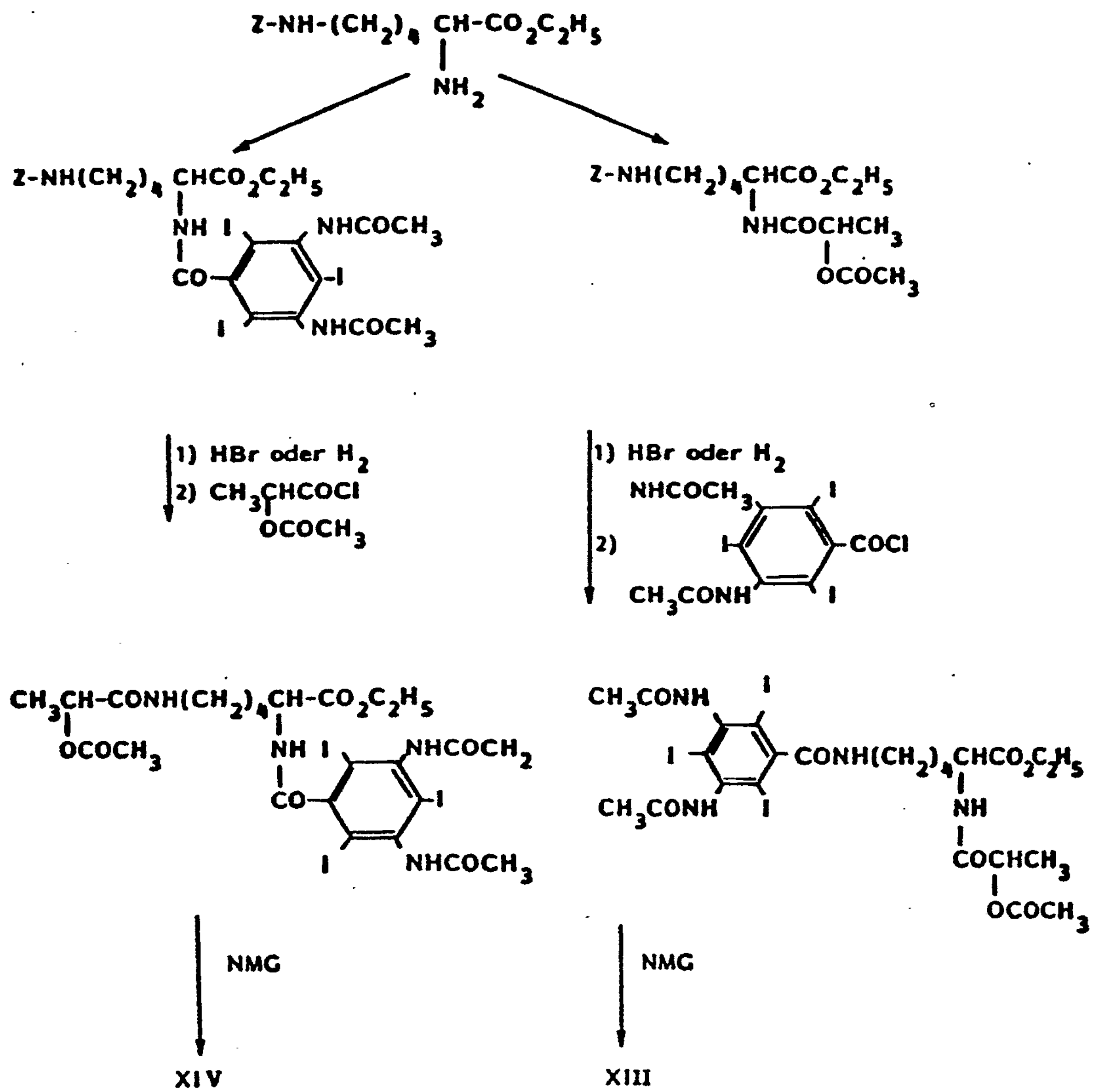


Z = $C_6H_5CH_2OCO$
NMG = N-Methylglucamin

Das Verfahren zur Herstellung der neuen Derivate gemäß der allgemeinen Formel I ist dadurch gekennzeichnet, daß man einen niedrigen Alkyl- oder Nitrophenylester der allgemeinen Formel XII mit N-Methylglucamin in einem kationsolvatisierenden Lösungsmittel in Gegenwart von Alkalicarbonat umsetzt (im Falle der Nitrophenylester ist kein Alkalicarbonat mehr notwendig). Als kationsolvatisierende Lösungsmittel werden vorzugsweise N,N-Dimethylformamid und N,N-Dimethylacetamid verwendet.

Als Alkalicarbonat wird Kaliumcarbonat am zweckmäßigsten verwendet.

Die Reaktionstemperatur ist von der Reaktivität des Esters (XII) abhängig. Niedrigere Temperaturen, beispielsweise 20 bis 35 °C sind für die reaktionsfähigen o- oder p-Nitrophenylester geeignet, während für die Alkylester (Methyl, Ethyl, Propyl, etc.) höhere Temperaturen, beispielsweise 60 bis 80 °C, verwendet werden müssen.

Die Reaktionsprodukte werden am zweckmäßigsten chromatographisch gereinigt.

Ein weiteres verfahren zur Herstellung der Verbindungen gemäß Formel I ist dadurch gekennzeichnet, daß man die entsprechenden N-(3,5-Diacetamido-2,4,6-triiodbenzoyl)-aminosäuren der allgemeinen Formel XI mit einem Alkylester der Chlorameisensäure in kationsolvatisierenden Lösungsmitteln in Gegenwart eines

tertiären Amins bei Temperaturen von -15 bis 0° C reagieren läßt. Anschließend setzt man das Anhydrid mit N-Methylglucamin in einem passenden Lösungsmittel wie N,N-Dimethylformamid, N,N-Dimethylacetamid, Ethanol, Wasser, bei Temperaturen von -10 bis +35° C um.
Als Ester der Chlorameisensäure werden Ethyl- oder Isobutylester bevorzugt. Als kationsolvatisierende Lösungsmittel werden N,N-Dimethylacetamid oder N,N-Dimethylformamid verwendet.
Das wichtigste Nebenprodukt dieser Methode ist die Ausgangssubstanz, die durch falsche Abspaltung des Anhydrids entsteht.

N-(3,5-Diacetamido-2,4,6-triiodbenzoyl)-aminosäurenethylester der allgemeinen Formel XII werden durch Umsetzung von 3,5-Diacetamido-2,4,6-triiodbenzoylchlorid mit den entsprechenden Aminosäurenethylestern in N,N-Dimethylformamid oder N,N-Dimethylacetamid in Gegenwart eines tertiären Amins bei Temperaturen von 20 - 60° C synthetisiert.

Dieses allgemeine Verfahren bezieht sich auch die Synthese von N-(3,5-Diacetamido-2,4,6-triiodbenzoyl)-L-asparaginsäureethylester (XXXI ) und -DL-asparaginsäureethylester (XXXII), obwohl beide Substanzen durch ein völlig anderes Verfahren schon 1963 synthetisiert wurden (Daiichi Seiyaku Co., Ltd., Japan 19. 101 ('65), Aug. 27, Appl. Sept. 23, 1963; Chemical Abstracts 63, 18260a, (1965)).

Als tertiäre Amine werden Triethylamin, Tributylamin oder Pyridin bevorzugt.

N-(3,5-Diacetamido-2,4,6-triiodbenzoyl)-aminosäuren der allgemeinen Formel XI werden durch alkalische Verseifung der entsprechenden Ester synthetisiert.

Dieses Verfahren wurde auch bei der Synthese von N-(3,5-Diacetamido-2,4,6-triiodbenzoyl)-L-asparaginsäure (XXXIII) und -DL-asparaginsäure (XXXIV) durchgeführt.

N-(3,5-Diacetamido-2,4,6-triiodbenzoyl)-aminosäuren-o(oder p-)-nitrophenylester der allgemeinen Formel XII werden durch Umsetzung von N-(3,5Diacetamido-2,4,6-triiodbenzoyl-aminosäuren mit o- (oder p-)-Nitrophenol in N,N-Dimethylformamid-Tetrahydrofuran-Gemisch in Gegenwart eines Kopplungsmittels, bei Temperaturen von 0 bis +30° C synthetisiert. Als Kopplungsmittel wird N,N-Dicyclohexylcarbodiimid bevorzugt.

Die zwei Lysinderivate werden folgendermaßen synthetisiert:
Man läßt ε,N-Benzyloxycarbonyl-lysinethylester mit 3,5-Diacetamido-2,4,6-triiodbenzoylchlorid, bzw. 0-Acetylmilchsäurechlorid in N,N-Dimethylformamid oder N,N-Dimethylacetamid in Gegenwart eines tertiären Amines, bei Temperaturen von 20-60° C reagieren.
Als tertiäres Amin wird Triethylamin oder Tributylamin bevorzugt.

Die Schutzgruppe von N-[ε-Benzyloxycarbonyl-α-(3,3-diacetamido-2,4,6-triiodbenzoyl)]-lysinethylester, bzw. N-[ε-Benzyloxycarbonyl-α-(0-acetyl-lactyl)]-lysinethylester wird mit Bromwasserstoff in Essigsäure oder mit Wasserstoff (Katalysator Palladium auf Aktivkohle) abgespaltet. Anschließend läßt man α,N-(3,5-Diacetamido-2,4,6-triiodbenzoyl)-lysinethylester, bzw. α,N-(0-Acetyl-lactyl)-lysinethylester mit 0-Acetylmilchsäurechlorid bzw. mit 3,5-Diacetamido-2,4,6-triiodbenzoylchlorid in N,N-Dimethylformamid oder N,N-Dimethylacetamid in Gegenwart eines tertiären Amines (Triethyl- oder Tributylamin), bei Temperaturen von 20-60° C reagieren.

Gegenstand der Erfindung ist auch ein Röntgenkontrastmittel auf der Basis der oben bezeichneten Endprodukte.

## Beispiele:

### Beispiel 1

#### N,N-[(3,5-Diacetamido-2,4,6-triiodbenzoyl)-sarkosin]-N-methylglucamid (II)

a) 2672 g (3,75 Mol) N-(3,5-Diacetamido-2,4,6-triiodbenzoyl)-sarkosinethylester, 1096 g (5,91 Mol) N-Methylglucamin und 264 g (1,91 Mol) Kaliumcarbonat in 6000 ml N,N-Dimethylformamid wurden 24 Std. bei 60° C gerührt (die Reaktion wurde chromatographisch verfolgt; DC-Kieselgel 60 $F_{254}$ n-Butanol-Essigsäure-Wasser 4 : 1 : 1). Das Reaktionsgemisch wurde auf Raumtemperatur abgekühlt, filtriert, der Niederschlag mit N,N-Dimethylformamid nachgewaschen, das Lösungsmittel unter Vakuum im Rotationsverdampfer eingedampft. Der Rückstand wurde mit einem Kationenaustauscher, dann mit einem Anionenaustauscher gerührt, die wäßrige Lösung unter Vakuum im Rotationsverdampfer konzentriert, über eine chromatographische Säule (Dowex 50 WX2, 100-200 mesh, $H^+$-Form, Elutionsmittel: Wasser) gereinigt. Die ausgewählten Fraktionen wurden mit einem Anionenaustauscher neutralisiert, mit Aktivkohle gerührt, filtriert, unter Vakuum im Rotationsverdampfer eingeengt, mit Methanol und Benzol gut getrocknet. Das Produkt wurde in N,N-Dimethylformamid gelöst und mit Essigester ausgefällt, abfiltriert, mit Essigester, dann mit Petroleumbenzin gewaschen über Phosphorpentoxid bei 70° C im Vakuumtrok-

kenschrank, dann über Paraffin bei Raumtemperatur getrocknet. Ausbeute: 1550 g (48 %).

**Analyse:** $C_{21}H_{29}N_4O_9I_3$ (862,19)
**Berechnet:** C 29.23 % H 3.36 % N 6.50 %
**Gefunden:** C 29.27 % H 3.52 % N 6.77 %

$\lambda_{Max.}^{H_2O}$ (log ε) : 240 nm (4,48)

Dünnschichtchromatographie (DC) Kieselgel $F_{254}$ (Merck) (Laufmittel (Lm): N-Butanol-Essigsäure-Wasser 4 : 1 : 1) $R_f$ = 0,25; 0,33

b) Zu einer auf -10 bis -15 °C abgekühlten Lösung von 6,85 g (10 mMol) N-(3,5-Diacetamido-2,4,6-triiodbenzoyl)-sarkosin in 15 ml N,N-Dimethylformamid werden 1,6 ml (11,6 mMol) Triethylamin, dann 1,1 ml (11,6 mMol) Chlorameisensäureethylester zugegeben. Nach 5 Minuten bei -10 bis -15 °C wird das Triethylammoniumchlorid abfiltriert und die Anhydrid-Lösung zu einer auf -10 °C abgekühlten Suspension von 2,15 g (12 mMol) N-Methylglucamin in 10 ml N,N-Dimethylformamid zugetropft. Gleich läßt man das Reaktionsgemisch auf Raumtemperatur erwärmen. Man rührt noch eine Stunde bei Raumtemperatur, dann wird das Reaktionsgemisch in 250 ml Essigester zugetropft und über Nacht bei Raumtemperatur gerührt. Der Niederschlag wird abfiltriert, mit Essigester, dann mit Petroleumbenzin gewaschen, getrocknet. Das Rohprodukt wird in Wasser gelöst und mit einem Kationenaustauscher, Anionenaustauscher, Aktivkohle gereinigt. Die farblose, wäßrige Lösung wird unter Vakuum eingeengt, azeotropisch getrocknet, in 15 ml N,N-Dimethylformamid gelöst und mit 150 ml Essigester präzipitiert. Das Produkt wird abfiltriert, gewaschen, getrocknet.
Ausbeute: 5 - 5,5 g (ca. 60 %).

c) 5,7 g (6,7 mMol) N-(3,5-Diacetamido-2,4,6-triiodbenzoyl)-sarkosin-o-nitrophenylester und 1,2 g (6.2 mMol) N-Methylglucamin in 15 ml N,N-Dimethylformamid werden 24 Std. bei Raumtemperatur gerührt. Das Reaktionsgemisch wird in 150 ml Essigester zugetropft, der Niederschlag abfiltriert, mit Essigester gut gewaschen, in Wasser gelöst, auf pH 3,5 eingestellt, mit Aktivkohle, dann mit einem Kationenaustauscher und einem Anionenaustauscher wie oben gereinigt und anschließend verarbeitet.
Ausbeute 2,7 g (ca. 50 %).

## Beispiel 2

### N,N-[(3,5-Diacetamido-2,4,6-triiodbenzoyl)-L-alanin]-N-methylglucamid (III)

Aus 48 g (0,067 Mol) N-(3,5-Diacetamido-2,4,6-triiodbenzoyl)-L-alaninethylester, 19,6 g (0,1 Mol) N-Methylglucamin, 4,7 g (0,034 Mol) Kaliumcarbonat und 480 ml N,N-Dimethylformamid werden analog Beispiel 1a) 35 g (61 %) des Endproduktes erhalten.

**Analyse:** $C_{21}H_{29}N_4O_9I_3$ (862,19)
**Berechnet:** C 29,25 % H 3,39 % N 6,50 %
**Gefunden:** C 29,32 % H 3,61 % N 6,65 %

$\lambda_{Max.}^{H_2O}$ (log ε) : 238 (4,49)

DC-Kieselgel, Lm (wie oben), $R_f$ = 0,33

## Beispiel 3

N,N-[(3,5-Diacetamido-2,4,6-triiodbenzoyl)-L-leucin]-N-methylglucamid (IV)

Aus 47,6 g (0,063 Mol) N-(3,5-Diacetamido-2,4,6-triiodbenzoyl)-L-leucinethylester, 19,5 g (0,1 Mol) N-Methylglucamin, 4,15 g (0,03 Mol) Kaliumcarbonat und 475 ml N,N-Dimethylformamid werden analog Beispiel 1a) 31 g (54 %) der gewünschten Substanz erhalten.

| Analyse: | $C_{24}H_{35}N_4O_9I_3$ | (904,27) | |
|---|---|---|---|
| Berechnet: | C 31,88 % | H 3,90 % | N 6,20 % |
| Gefunden: | C 31,66 % | H 4,31 % | N 5,94 % |

$\lambda_{Max.}^{H_2O}$ (log ε) : 238 (4,36)

DC-Kieselgel, Lm (wie oben), $R_f$ = 0,48

Beispiel 4

N,N-[(3,5-Diacetamido-2,4,6-triiodbenzoyl)-L-serin]-N-methylglucamid (V)

Analog Beispiel 1a) werden 187 g (55 %) der gewünschten Verbindung aus 280 g (0,385 Mol) N-(3,5-Diacetamido-2,4,6-triiodbenzoyl)-L-serinethylester, 112 g (0,574 Mol) N-Methylglucamin, 26,5 g (0,192 Mol) Kaliumcarbonat und 2800 ml N,N-Dimethylformamid synthetisiert.

| Analyse: | $C_{21}H_{29}N_4O_{10}I_3$ | (878,19) | |
|---|---|---|---|
| Berechnet: | C 28,72 % | H 3,33 % | N 6,38 % |
| Gefunden: | C 29,38 % | H 3,55 % | N 6,65 % |

$\lambda_{Max.}^{H_2O}$ (log ε) : 238 (4,46)

DC-Kieselgel, Lm (wie oben), $R_f$ = 0,26

Beispiel 5

N,N-[(3,5-Diacetamido-2,4,6-triiodbenzoyl)-L-asparaginsäure]-bis-N-methylglucamid (VI)

Aus 133 g (0,17 Mol) N-(3,5-Diacetamido-2,4,6-triiodbenzoyl)-L-asparaginsäurediethylester, 330 g (1,7 Mol) N-Methylglucamin und 25,5 g (0,185 Mol) Kaliumcarbonat in 1300 ml N,N-Dimethylformamid werden analog Beispiel 1a) 140 g (75 %) des Endproduktes hergestellt.

| Analyse: | $C_{29}H_{44}N_5O_{15}I_3$ | (1083,40) | |
|---|---|---|---|
| Berechnet: | C 32,15 % | H 4,09 % | N 6,46 % |
| Gefunden: | C 32,06 % | H 4,53 % | N 6,60 % |

$\lambda_{Max.}^{H_2O}$ (log ε) : 238 (4,44)

DC-Kieselgel, Lm (wie oben), $R_f$ = 0,09

Beispiel 6

N,N-[(3,5-Diacetamido-2,4,6-triiodbenzoyl)-DL-asparaginsäure]-bis-N-methylglucamid (VII)

Aus 70,5 g (0,09 Mol) N-(3,5-Diacetamido-2,4,6-triiodbenzoyl)-DL-asparaginsäurediethylester, 52,5 g (0,27 Mol) N-Methylglucamin, 6 g (0,043 Mol) Kaliumcarbonat und 700 ml N,N-Dimethylformamid werden analog Beispiel 1 a) 65,5 g (64 %) dieser Substanz synthetisiert.

| | | | |
|---|---|---|---|
| Analyse: | $C_{29}H_{44}N_5O_{15}I_3 \cdot 3\,H_2O$ (1137,44) | | |
| Berechnet: | C 30,62 % | H 4,43 % | N 6,16 % |
| Gefunden: | C 29,92 % | H 4,66 % | N 6,34 % |

$\lambda^{H_2O}_{Max.}$ (log ε) : 238 (4,46)

DC-Kieselgel, Lm (wie oben), $R_f$ = 0,08

Beispiel 7

N,N-[(3,5(-Diacetamido-2,4,6-triiodbenzoyl)-L-glutaminsäure]-bis-N-methylglucamid (VIII)

Aus 12,3 g (0,015 Mol) N-(3,5-Diacetamido-2,4,6-triiodbenzoyl)-L-glutaminsäurediethylester, 30 g (0,154 Mol) N-Methylglucamin, 2,35 g (0,017 Mol) Kaliumcarbonat und 150 ml N,N-Dimethylformamid werden analog Beispiel 1 a) 12 g (70 %) der gewünschten Verbindung erhalten.

| | | | |
|---|---|---|---|
| Analyse: | $C_{30}H_{46}N_5O_{15}I_3$ (1097,43) | | |
| Berechnet: | C 32,83 % | H 4,22 % | N 6,38 % |
| Gefunden: | C 33,61 % | H 4,72 % | N 6,47 % |

$\lambda^{H_2O}_{Max.}$ (log ε) : 238 (4,42)

DC-Kieselgel, Lm (wie oben), $R_f$ = 0,07

Beispiel 8

N,N-[α-Lactyl-ε-(3,5-diacetamido-2,4,6-triiodbenzoyl)-L-lysin]-N-methyl-glucamid (IX)

Aus 37,8 g (0,425 Mol) N- [α-(0-Acetyl-lactyl)-ε-(3,5-diacetamido-2,4,6-triiodbenzoyl)]-L-lysinethylester, 12,5 g (0,064 Mol) N-Methylglucamin und 3 g (0,0217 Mol) Kaliumcarbonat werden analog Beispiel 1 a) ca. 8 g (20 %) dieser Substanz erhalten.

| | | | |
|---|---|---|---|
| Analyse: | $C_{27}H_{40}N_5O_{11}I_3$ (991,35) | | |
| Berechnet: | C 32,71 % | H 4,07 % | N 7,07 % |
| Gefunden: | C 33,60 % | H 4,05 % | N 6,79 % |

$\lambda^{H_2O}_{Max.}$ (log ε) : 238 (4,47)

DC-Kieselgel, Lm (wie oben), $R_f$ = 0,33

Beispiel 9

N,N-[ε-Lactyl-α-(3,5-diacetamido-2,4,6-triiodbenzoyl)-L-lysin-N-methylglucamid (X)

Aus 22, 2 g (0,025 Mol) N-[ε-(0-Acetyl-lactyl)-α-(3,5-diacetamido-2,4,6-triiodbenzoyl)]-L-lysinethylester, 7,5 g (0,0385 Mol) N-Methylglucamin und 1,8 g (0,013 Mol) Kaliumcarbonat werden analog Beispiel 1 a) ca. 7 g (29 %) des Endproduktes synthetisiert.

| Analyse: | $C_{27}H_{40}N_5O_{11}I_3$ (991,35) | | |
|---|---|---|---|
| Berechnet: | C 32,71 % | H 4,07 % | N 7,07 % |
| Gefunden: | C 33,60 % | H 4,30 % | N 7,12 % |

$\lambda_{Max.}^{H_2O}$ (log ε) : 238 (4,47)

DC-Kieselgel, Lm (wie oben), $R_f$ = 0,27

Beispiel 10

N- (3,5-Diacetamido-2,4,6-triiodbenzoyl)-sakosinethylester (XIV)

Zu einer Lösung von 759 g (1,2 Mol) 3,5-Diacetamido-2,4,6-triiodbenzoylchloridund 276 g (1,8 Mol) Sarkosinethylesterhydrochlorid in 7600 ml N,N-Dimethylformamid werden 840 ml (6,1 Mol) Triethylamin zugegeben und 24 Std. bei 60 °C gerührt. Das Reaktionsgemisch wird auf Raumtemperatur abgekühlt, das auskristallisierte Triethylammoniumchlorid abgesaugt, mit N,N-Dimethylformamid gewaschen, im Rotationsverdampfer unter Vakuum eingeengt. Der Rückstand wird in 650 ml Methanol gelöst und in 3 l Essigester zugetropft. Der Niederschlag wird über Nacht auskristallisieren lassen, abgesaugt, gut trockensaugen lassen und anschließend mit Wasser, 1 N-Salzsäure, kaltgesättigter Natriumhydrogencarbonat-Lösung, Wasser gewaschen, im Trockenschrank bei 60 °C getrocknet. Ausbeute 685 g (80 %).

| Analyse: | $C_{16}H_{18}N_3O_5I_3$ (713,05) | | |
|---|---|---|---|
| Berechnet: | C 26,95 % | H 2,55 % | N 5,89 % |
| Gefunden: | C 26,69 % | H 2,40 % | N 5,70 % |

Beispiel 11

N-(3,5-Diacetamido-2,4,6-triiodbenzoyl)-L-alaninester (XVII)

Aus 63,2 g (0.1 Mol) 3, 5-Diacetamido-2,4,6-triiodbenzoylchlorid und 23,1 g (0,15 Mol) L-Alaninethylesterhydrochlorid werden analog Beispiel 10 50 g (70 %) der gewünschten Substanz gewonnen.

| Analyse: | $C_{16}H_{18}N_3O_5I_3$ (713,05) | | |
|---|---|---|---|
| Berechnet: | C 26,95 % | H 2,55 % | N 5,89 % |
| Gefunden: | C 27,62 % | H 2,56 % | N 5,87 % |

Beispiel 12

N-(3,5-Diacetamido-2,4,6-triiodbenzoyl)-L-leucinethylester (XX)

48 g (64 %) dieses Produkts wurden analog Beispiel 10 aus 63,2 g (0,1 Mol) 3,5-Diacetamido-2,4,6-triiodbenzoylchlorid und 29,4 g (0,15 Mol) L-Leucinethylesterhydrochlorid synthetisiert.

| | | | | |
|---|---|---|---|---|
| **Analyse:** | $C_{19}H_{24}N_3O_5I_3$ | (755,03) | | |
| **Berechnet:** | C 30,21 % | H 3,20 % | N 5,56 % | |
| **Gefunden:** | C 30,33 % | H 3,22 % | N 5,46 % | |

Beispiel 13

N-(3,5-Diacetamido-2,4,6-triiodbenzoyl)-L-serinethylester (XXIII)

Aus 295 g (0,47 Mol) 3,5-Diacetamido-2,4,6-triiodbenzoylchlorid und 119 g (0,7 Mol) L-Serinethylesterhydrochlorid werden analog Beispiel 10 281 g (82 %) des Produkts erhalten

| | | | | |
|---|---|---|---|---|
| **Analyse:** | $C_{16}H_{17}N_3O_6I_3$ | (728,04) | | |
| **Berechnet:** | C 26,36 % | H 2,50 % | N 5,76 % | |
| **Gefunden:** | C 25,90 % | H 2,27 % | N 5,65 % | |

Beispiel 14

N-(3,5-Diacetamido-2,4,6-triiodbenzoyl)-L-asparaginsäurediethylester (XXXI)

Analog Beispiel 10 werden aus 140 g (0,22 Mol) 3,5-Diacetamido-2,4,6-triiodbenzoylchlorid und 75 g (0,33 Mol) L-Asparaginsäurediethylesterhydrochlorid 112 g (65 %) des Endproduktes synthetisiert.

| | | | | |
|---|---|---|---|---|
| **Analyse:** | $C_{19}H_{22}N_3O_7I_3$ | (785,01) | | |
| **Berechnet:** | C 29,06 % | H 2,82 % | N 5,35 % | |
| **Gefunden:** | C 29,44 % | H 2,86 % | N 5,11 % | |

Beispiel 15

N-(3,5-Diacetamido-2,4,6-triiodbenzoyl)-DL-asparaginsäurediethyester (XXXII)

Aus 127 g (0,2 Mol) 3,5-Diacetamido-2,4,6-triiodbenzoylchlorid und 67,5 g (0,3 Mol) DL-Asparaginsäurediethylesterhydrochlorid werden analog Beispiel 10 ca. 100 g (65 %) der gewünschten Verbindung erhalten.

| | | | | |
|---|---|---|---|---|
| **Analyse:** | $C_{19}H_{22}N_3O_7I_3$ | (785,01) | | |
| **Berechnet:** | C 29,06 % | H 2,82 % | N 5,35 % | |
| **Gefunden:** | C 29,04 % | H 2,95 % | N 5,39 % | |

Beispiel 16

N-(3,5-Diacetamido-2,4,6-triiodbenzoyl)-L-glutaminsäurediethylester (XXVI)

Aus 192 g (0,3 Mol) 3,5-Diacetamido-2,4,6-triiodbenzoylchlorid und 108 g (0,45 Mol) L-Glutaminsäurediethylesterhydrochlorid werden analog Beispiel 10 155 g (65 %) dieses Produkts synthetisiert.

| | | | |
|---|---|---|---|
| **Analyse:** | $C_{20}H_{24}N_3O_7I_3$ (799,04) | | |
| **Berechnet:** | C 30,05 % | H 3,03 % | N 5,30 % |
| **Gefunden:** | C 30,11 % | H 3,02 % | N 5,32 % |

Beispiel 17

N-(3,5-Diacetamido-2,4,6-triiodbenzoyl)-sarkosin (XIII)

Zu einer auf 0° C abgekühlten Suspension von 890 g (1,25 Mol) N-(3,5-Diacetamido-2,4,6-triiodbenzoyl)-sarkosinethylester in 3 l Methanol werden 1375 ml 1 N Natriumhydroxid zugetropft, so daß die Temperatur nicht über +7° C steigt. Nach 2 Stunden bei 0° C und über Nacht bei Raumtemperatur wird die Lösung angesäuert (pH 2,5; 6N HCl). Bald beginnt ein Niederschlag auszuscheiden. Man rührt weiter einige Stunden bei Raumtemperatur, dann wird stark abgekühlt (Eis-Salz-Gemisch), das Präzipitat abfiltriert, mit etwas Wasser gewaschen, getrocknet. Ausbeute 668 g (77 %).

| | | | |
|---|---|---|---|
| **Analyse:** | $C_{14}H_{14}N_3O_5I_3 \cdot H_2O$ (703,01) | | |
| **Berechnet:** | C 23,92 % | H 2,29 % | N 5,98 % |
| **Gefunden:** | C 24,14 % | H 2,46 % | N 5,78 % |

Beispiel 18

N-(3,5-Diacetamido-2,4,6-triiodbenzoyl)-L-alanin (XVI)

Aus 71,3 g (0,1 Mol) N-(3,5-Diacetamido-2,4,6-triiodbenzoyl)-L-alaninethylester werden analog Beispiel 17 65 g (92 %) dieser Substanz synthetisiert.

| | | | |
|---|---|---|---|
| **Analyse:** | $C_{14}H_{14}N_3O_5I_3 \cdot 3\ H_2O$ (739,04) | | |
| **Berechnet:** | C 22,75 % | H 2,72 % | N 5,69 % |
| **Gefunden:** | C 22,74 % | H 2,05 % | N 5,71 % |

Beispiel 19

N-(3,5-Diacetamido-2,4,6-triiodbenzoyl)-L-leucin (XIX)

Aus 75,5 g (0,1 Mol) N-(3,5-Diacetamido-2,4,6-triiodbenzoyl)-L-leucinethylester werden analog Beispiel 17 62 g (85 %) dieser Verbindung erhalten.

**Analyse:** $C_{17}H_{20}N_3O_5I_3$ (727,07)
**Berechnet:** C 28,08 % H 2,77 % N 5,78 %
**Gefunden:** C 28,05 % H 3,00 % N 5,40 %

Beispiel 20

N-(3,5-Diacetamido-2,4,6-triiodbenzoyl)-L-serin (XXII)

Aus 72,8 g (0,1 Mol) N-(3,5-Diacetamido-2,4,6-triiodbenzoyl)-L-serinethylester werden analog Beispiel 17 56 g (80 %) des Endprodukts synthetisiert.

**Analyse:** $C_{14}H_{14}N_3O_6I_3$ (700,99)
**Berechnet:** C 23,99 % H 2,01 % N 5,99 %
**Gefunden:** C 24,62 % H 2,10 % N 5,95 %

Beispiel 21

N-(3,5-Diacetamido-2,4,6-triiodbenzoyl)-L-asparaginsäure (XXXIII)

Aus 618 g (0,787 Mol) N-(3,5-Diacetamido-2,4,6-triiodbenzoyl)-L-asparaginsäurediethylester werden analog Beispiel 17 455 g (80 %) des gewünschten Produkts erhalten.

**Analyse:** $C_{15}H_{14}N_3O_7I_3 \cdot 2\ H_2O$ (765,03)
**Berechnet:** C 23,55 % H 2,37 % N 5,49 %
**Gefunden:** C 22,84 % H 1,84 % N 5,46 %

Beispiel 22

N-(3,5-Diacetamido-2,4,6-triiodbenzoyl)-DL-asparaginsäure (XXXIV)

Aus 78,5 g (0,1 Mol) N-(3,5-Diacetamido-2,4,6-triiodbenzoyl)-DL-asparaginsäurediethylester werden analog Beispiel 17 58 g (80 %) dieser Verbindung synthetisiert.

**Analyse:** $C_{15}H_{14}N_3O_7I_3 \cdot 3\ H_2O$ (783,04)
**Berechnet:** C 23,01 % H 2,57 % N 5,37 %
**Gefunden:** C 22,37 % H 2,18 % N 5,46 %

Beispiel 23

N- (3,5-Diacetamido-2,4,6-triiodbenzoyl)-L-glutaminsäure (XXV)

Aus 192,6 g (0,24 Mol) N-(3,5-Diacetamido-2,4,6-triiodbenzoyl)-L-glutaminsäurediethylester werden analog Beispiel 17 170,7 g (95 %) der gewünschten Substanz hergestellt.

| | | | |
|---|---|---|---|
| **Analyse:** | $C_{16}H_{16}N_3O_7I_3$ (743,03) | | |
| **Berechnet:** | C 25,86 % | H 2,17 % | N 5,65 % |
| **Gefunden:** | C 25,00 % | H 2,18 % | N 5,60 % |

Beispiel 24

N-(3,5-Diacetamido-2,4,6-triiodbenzoyl)-sarkosin-o-nitrophenylester (XV)

6,85 g (10 mMol) N-(3,5-Diacetamido-2,4,6-triiodbenzoyl)-sarkosin und 1,7 g (12,2 mMol) o-Nitrophenol werden in 15 ml N,N-Dimethylformamid gelöst. Zu dieser Lösung gibt man 60 ml Tetrahydrofuran, dann wird sie auf 0° C abgekühlt. Es wird 2,5 g (12 mMol) N,N-Dicyclohexylcarbodiimid hinzugefügt. Die Reaktion läuft 2 Stunden bei 0° C und über Nacht bei Raumtemperatur. Der Niederschlag (N,N-Dicyclohexylharnstoff) wird abfiltriert, mit 10 ml N,N-Dimethylformamid gewaschen, Tetrahydrofuran in Vakuum abdestilliert, über Nacht im Kühlschrank aufbewahrt, Dicyclohexylharnstoff wieder filtriert Die organische Lösung wird in 250 ml Diethylester zugetropft, der Niederschlag abdekantiert, mit 25 ml N,N-Dimethylformamid gelöst und in 250 ml Wasser zugetropft. Das Präzipitat wird abgesaugt, mit gesättigter Natriumhydrogencarbonat-Lösung und Wasser gewaschen, getrocknet. Ausbeute: 5,4 g (67 %)

| | | | |
|---|---|---|---|
| **Analyse:** | $C_{20}H_{17}N_4O_7I_3$ (806,09) | | |
| **Berechnet:** | C 29,80 % | H 2,13 % | N 6,95 % |
| **Gefunden:** | C 30,07 % | H 2,64 % | N 6,66 % |

Beispiel 25

N-(3,5-Diacetamido-2,4,6-triiodbenzoyl)-L-alanin-o-nitrophenylester (XVIII)

Aus 13,7 g (20 mMol) N-(3,5-Diacetamido-2,4,6-triiodbenzoyl)-L-alanin, 3,5 g (25 mMol) o-Nitrophenol und 5 g (24 mMol) N,N-Dicyclohexylcarbodiimid wird analog Beispiel 24 die gewünschte Substanz synthetisiert.

| | | | |
|---|---|---|---|
| **Analyse:** | $C_{20}H_{17}N_4O_7I_3$ (806,09) | | |
| **Berechnet:** | C 29,80 % | H 2,13 % | N 6,95 % |
| **Gefunden:** | C 30,49 % | H 2,46 % | N 6,86 % |

Beispiel 26

N-(3,5-Diacetamido-2,4,6-triiodbenzoyl)-L-leucin-o-nitrophenylester (XXI)

Analog Beispiel 24 wird das Endprodukt aus 14,6 g (20 mMol) N-(3,5-Diacetamido-2,4,6-triiodbenzoyl)-L-leucin, 3,5 g (25 mMol) o-Nitrophenol und 5 g (24 mMol) N,N-Dicyclohexylcarbodiimid synthetisiert. Ausbeute: 12,5 g (73 %).

**Analyse:** $C_{23}H_{23}N_4O_7I_3$ (848,17)
**Berechnet:** C 32,57 % H 2,73 % N 6,61 %
**Gefunden:** C 32,39 % H 3,27 % N 6,37 %

Beispiel 27

N-(3,5-Diacetamido-2,4,6-triiodbenzoyl)-L-serin-o-nitrophenylester (XXIV)

Aus 14 g (20 mMol) N-(3,5-Diacetamido-2,4,6-triiodbenzoyl)-L-serin, 3,5 g (25 mMol) o-Nitrophenol und 5 g (24 mMol) N,N-Dicyclohexylcarbodiimid werden analog Beispiel 24 ca. 8,5 g (52 %) der gewünschten Verbindung synthetisiert.

**Analyse:** $C_{20}H_{17}N_4O_8I_3$ (822,09)
**Berechnet:** C 29,22 % H 2,08 % N 6,81 %
**Gefunden:** C 30,14 % H 2,27 % N 6,86 %

Beispiel 28

N-[ε -Benzoyloxycarbonyl-α-(3,5-diacetamido-2,4,6-triiodbenzoyl]-L-lysinethylester (XXIX)

39 g (0,127 Mol) ε,N-Benzyloxycarbonyl-L-lysinethylester, 80,1 g (0,126 Mol) 3,5-Diacetamido-2,4,6-triiodbenzoylchlorid und 26,5 ml (0,19 Mol) Triethylamin in 800 ml N,N-Dimethylformamid werden 24 Std. bei 60° C gerührt, dann das Lösungsmittel unter Vakuum abdestilliert, der Rückstand mit Essigester versetzt, auskristallisieren lassen, der Niederschlag abfiltriert, mit Petroleumbenzin gewaschen, trockensaugen lassen, mit Wasser, 1 N Salzsäure, Wasser, kaltgesättigter Natriumhydrogencarbonat-Lösung und Wasser gewaschen, getrocknet. Ausbeute: 86 g (75 %).

**Analyse:** $C_{27}H_{31}N_4O_7I_3$ (904,28)
**Berechnet:** C 35,86 % H 3,46 % N 6,20 %
**Gefunden:** C 36,05 % H 3,41 % N 6,19 %

Beispiel 29

N-[ε -Benzyloxycarbonyl-α-(O-acetyl-lactyl]-L-lysinethylester (XXX)

43,4 g (0,14 Mol) ε,N-Benzyloxycarbonyl-L-lysinethylester und 30 ml (0,217 Mol) Triethylamin werden in 430 ml Essigester gelöst und auf 0° C abgekühlt. Bei dieser Temperatur werden 24 g (0,16 Mol) O-Acetylmilchsäurechlorid in 60 ml Essigester zugetropft. Die Reaktion läuft 2 Stunden bei 0° C und über Nacht bei Raumtemperatur. Am nächsten Tag wird das Triethylammoniumchlorid filtriert, die organische Lösung mit Wasser, gesättigter Natriumhydrogencarbonat-Lösung und Wasser gewaschen, über Natriumsulfat getrocknet, das Lösungsmittel abdestilliert. Ausbeute: 51,8 g (86 %).

| | | | | |
|---|---|---|---|---|
| **Analyse:** | $C_{21}H_{30}N_2O_7$ (422,48) | | | |
| **Berechnet:** | C 59,70 % | H 7,16 % | N 6,63 % | |
| **Gefunden:** | C 59,31 % | H 7,22 % | N 6,45 % | |

Beispiel 30

N-[ε -(O-Acetyl-lactyl)-α-(3,5-diacetamido-2,4,6-triiodbenzoyl)]-L-lysinethylester (XXVIII)

42,6 g (0,047 Mol) N-[ε-Benzyloxycarbonyl-α-(3,5-diacetamido-2,4,6-triiodbenzoyl)]-L-lysinethylester werden in 235 ml Essigsäure suspendiert und mit 50 ml 33 % Bromwasserstoff in Essigsäure versetzt. Nach 2 standen bei Raumtemperatur wird die Lösung in 1,5 l Diethylether zugetropft, der Niederschlag abfiltriert, mit Diethylether gewaschen. Das Hydrobromid wird in 250 ml N,N-Dimethylformamid gelöst, mit 13,8 ml (0,1 Mol) Triethylamin versetzt, über Nacht bei Raumtemperatur gerührt, das Triethylammoniumbromid filtriert, das Lösungsmittel abdestilliert. Der Rückstand (ca. 35 g) wird in 175 ml N,N-Dimethylformamid gelöst, mit 7,8 g (0,060 Mol) 0-Acetylmilchsäurechlorid und 13,8 ml (0.1 Mol) Triethylamin versetzt, 24 Stunden bei Raumtemperatur gerührt, Triethylammoniumchlorid abfiltriert, N,N-Dimethylformamid abdestilliert, der ölige Rückstand mit Essigester versetzt. Bald kristallisiert die Substanz aus. Sie wird abfiltriert, mit Petroleumbenzin gewaschen, trockensaugen lassen, mit Wasser gewaschen, getrocknet. Ausbeute: 30,4 g (75 %).

| | | | | |
|---|---|---|---|---|
| **Analyse:** | $C_{24}H_{31}N_4O_8I_3$ (884,24) | | | |
| **Berechnet:** | C 32,60 % | H 3,53 % | N 6,34 % | |
| **Gefunden:** | C 32,66 % | H 3.64 % | N 6.16 % | |

Beispiel 31

N-[α-(O-Acetyl-lactyl)-ε-(3,5-Diacetamido-2,4,6-triiodbenzoyl)]-L-lysinethylester (XXVII)

51,8 g (0,12 Mol) N-ε-Benzyloxycarbonyl-α-(0-acetyl-lactyl)]-L-lysinethylester werden in 150 ml Essigsäure gelöst und unter Rühren 120 ml 33 % Bromwasserstoff in Essigsäure zugetropft. Nach 2 Stunden bei Raumtemperatur wird die Lösung in 1,5 l Diethylether zugetropft, nach einigen Stunden abdekantiert, das Öl in 500 ml N,N-Dimethylformamid gelöst, mit 35 ml (0,25 Mol) Triethylamin versetzt, über Nacht gerührt, das Triethylammoniumbromid abfiltriert, das Lösungsmittel unter Vakuum abdestilliert. Der Rückstand wird in 450 ml N,N-Dimethylformamid gelöst, 75 g (0,12 Mol) 3, 5-Diacetamido-2,4,6-triiodbenzoylchlorid zugegeben, dann mit 35 ml (0,25 Mol) Triethylamin versetzt. Die Reaktion läuft 24 Stunden bei Raumtemperatur. Am nächsten Tag wird das Triethylammoniumchlorid abfiltriert, das Lösungsmittel unter Vakuum abdestilliert. Der Rückstand wird mit Essigester versetzt. Die auskristallisierte Substanz wird abfiltriert, mit Petroleumbenzin gewaschen, trockensaugen lassen, mit Wasser, 1 N Salzsäure, Wasser, gesättigter Natriumhydrogencarbonat-Lösung und Wasser gewaschen, getrocknet. Ausbeute: 45 g (42 %).

| | | | | |
|---|---|---|---|---|
| **Analyse:** | $C_{24}H_{31}N_4O_8I_3$ (884,24) | | | |
| **Berechnet:** | C 32,60 % | H 3,53 % | N 6,34 % | |
| **Gefunden:** | C 32,58 % | H 3,15 % | N 6,17 % | |

**Patentansprüche**

1. N,N-[(3,5-Diacetamido-2,4,6-triiodbenzoyl)-aminoacyl]-N-methylglucamid der allgemeinen Formel

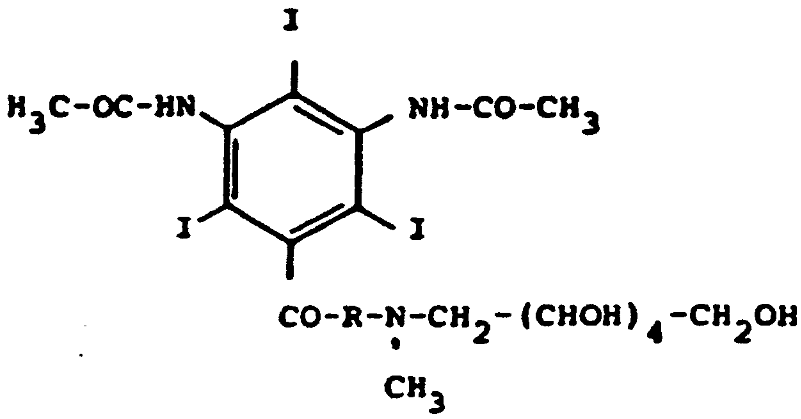

(I)

worin R einen Alanin-, Sarkosin-, Leucin-, Serin-, N-Lactyl-lysin-, Asparaginsäure-, Glutaminsäurerest bedeutet.

2. N,N-[(3,5-Diacetamido-2,4,6-triiodbenzoyl)-sarkosin]-N-methylglucamid(II).

3. N,N-[(3,5-Diacetamido-2,4,6-triiodbenzoyl)-L-alanin]-N-methylglucamid (III).

4. N,N-[(3,5-Diacetamido-2,4,6-triiodbenzoyl)-L-leucin]-N-methylglucamid (IV).

5. N,N-[(3,5-Diacetamido-2,4,6-triiodbenzoyl)-L-serin]-N-methylglucamid (V).

6. N,N-[(3,5-Diacetamido-2,4,6-triiodbenzoyl)-L-asparaginsäure]-bis-N-methylglucamid (VI).

7. N,N-[(3,5-Diacetamido-2,4,6-triiodbenzoyl)-DL-asparaginsäure]-bis-N-methylglucamid (VII).

8. N,N-[(3,5-Diacetamido-2,4,6-triiodbenzoyl)-L-glutaminsäure]-bis-N-methylglucamid (VIII).

9. N,N-[α-Lactyl-ε-(3,5-diacetamido-2,4,6-triiodbenzoyl)-lysin]-N-methylglucamid (IX).

10. N,N-[ε -Lactyl-α-(3,5-diacetamido-2,4,6-triiodbenzoyl)-lysin]-N-methylglucamid (X).

11. N-(3,5-Diacetamido-2,4,6-triiodbenzoyl)-aminosäuren der allgemeinen Formel XI

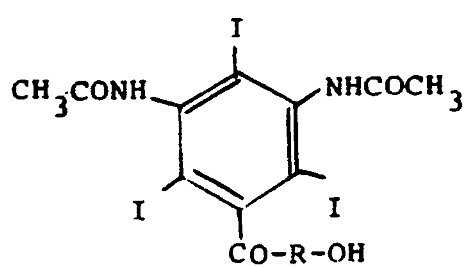

worin R einen Alanin-, Sarkosin-, Leucin-, Serin-, N-Lactyl-lysin-, Asparaginsäure-, Glutaminsäurerest bedeutet, und N-(3,5-Diacetamido-2,4,6-triiodbenzoyl)-aminosäurenester der allgemeinen Formel XII

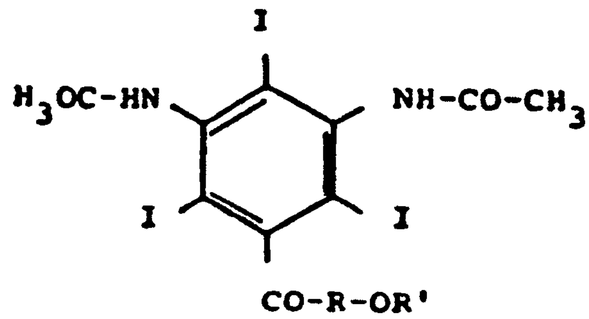

worin R einen Alanin-, Sarkosin-, Leucin-, Serin-, N-Lactyl-lysin-, Asparaginsäure, Glutaminsäurerest bedeutet und R' ein Alkyl- oder o- oder p-Nitrophenylester ist.

12. N-(3,5-Diacetamido-2,4,6-triiodbenzoyl)-sarkosin (XIII), -sarkosinethylester (XIV), -sarkosin-o-nitrophenylester (XV).

13. N-(3,5-Diacetamido-2,4,6-triiodbenzoyl)-L-alanin (XVI), -L-alaninethylester (XVII), -L-alanin-o-nitrophenylester (XVIII).

14. N-(3,5-Diacetamido-2,4,6-triiodbenzoyl)-L-leucin (XIX), -L-leucinethylester (XX), -L-leucin-o-nitrophenylester (XXI).

15. N-(3,5-Diacetamido-2,4,6-triiodbenzoyl)-L-serin (XXII), -L-serinethylester (XXIII), -L-serin-o-nitrophenylester (XXIV).

16. N-(3,5-Diacetamido-2,4,6-triiodbenzoyl)-L-glutaminsäure (XXV), -L-glutaminsäurediethylester (XXVI).

17. N-[α-(O-Acetyl-lactyl)-ε-(3,5-diacetamido-2,4,6-triiodbenzoyl)]-L-lysinethylester (XXVII).

18. N-[ε-(O-Acetyl-lactyl)-α-(3,5-diacetamido-2,4,6-triiodbenzoyl)]-L-lysinethylester (XXVIII).

19. N-[ε-Benzyloxycarbonyl-α-(3,5-diacetamido-2,4,6-triiodbenzoyl)]-L-lysinethylester (XXIX).

20. N-[ε-Benzyloxycarbonyl-α-(O-acetyl-lactyl)]-L-lysinethylester (XX) mit der Formel

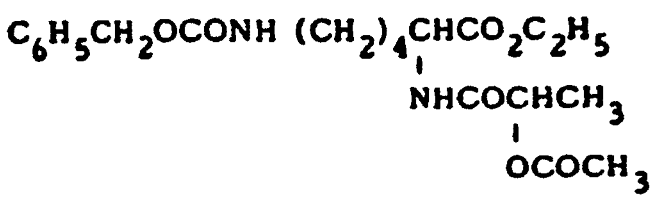

(XX)

21. Verfahren zur Herstellung von N,N-[(3,5-Diacetamido-2,4,6-triiodbenzoyl)-aminoacyl]-N-methylglucamid der Formel

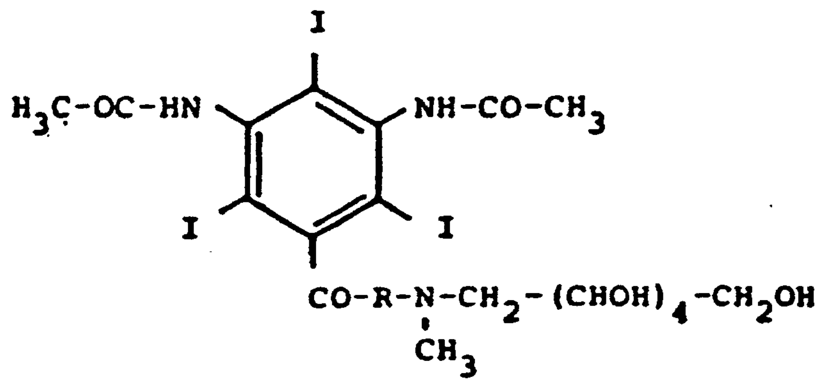

(I)

gemäß Anspruch 1, dadurch **gekennzeichnet,** daß man einen Ester der allgemeinen Formel

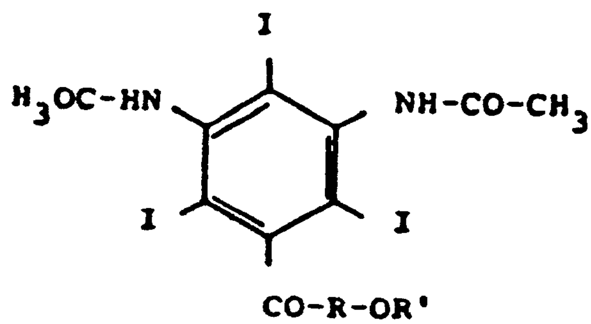

(XII)

worin R einen Alanin-, Sarkosin-, Leucin-, Serin-, N-Lactyl-lysin-, Asparaginsäure-, Glutaminsäurerest bedeutet und R' ein Alkyl- oder o- oder p-Nitrophenyl-Esterrest ist, in an sich bekannter Weise mit N-Methylglucamin in einem kationsolvierenden Lösungsmittel, wie N,N-Dimethylformamid, N,N-Dimethylacetamid oder Dimethylsulfoxid in Gegenwart eines Alkalicarbonats, beispielsweise bei Temperaturen von 20 bis 80 °C, reagieren läßt.

**22.** Verfahren zur Herstellung von Verbindungen der allgemeinen Formel I, dadurch **gekennzeichnet,** daß man Säuren der allgemeinen Formel

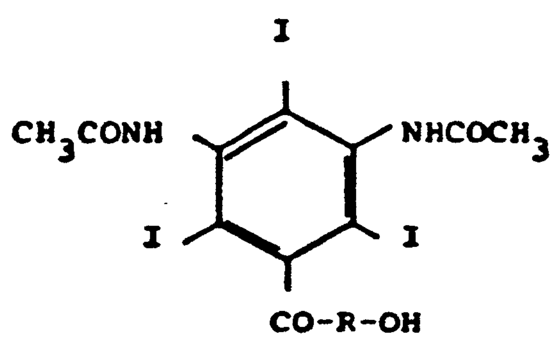

(XI)

worin R einen Alanin-, Sarkosin-, Leucin-, Serin-, N-Lactyl-lysin-, Asparaginsäure-, Glutaminsäurerest bedeutet, mit einem niedrigen Alkylester von Chlorameisensäure in einem kationsolvatisierenden Lösungsmittel wie N,N-Dimethylformamid oder N,N-Dimethylacetamid in Gegenwart eines tertiären Amins wie Triethylamin oder 4-Methylmorpholin bei Temperaturen von -15 bis 0 °C umsetzt und anschließend das Anhydrid mit N-Methylglucamin in einem Lösungsmittel wie N,N-Dimethylformamid, N,N-Dimethylacetamid, Methanol, Ethanol oder wasser bei Temperaturen von -10 bis +35 °C reagieren läßt.

23. Verfahren zur Herstellung von N-(3,5-Diacetamido-2,4,6-triiodbenzoyl-, Alanin-, Sarkosin-, Leucin-, Serin-, N-Lactyl-lysin-, Asparaginsäure-, Glutaminsäureethylester, dadurch **gekennzeichnet,** daß man 3,5-Diacetamido-2,4,6-triiodbenzoylchlorid mit Glycin-, Alanin-, ß-Alanin-, Sarkosin-, Aminobuttersäure-, Leucin-, Serin-, N-Lactyl-lysin-, Asparaginsäure-, Glutaminsäureethylestern in N,N-Dimethylformamid oder N,N-Dimethylacetamid in Gegenwart eines tertiären Amins wie Triethylamin, Tributylamin oder Pyridin bei Temperaturen von 20 bis 60 °C reagieren läßt.

24. Verfahren zur Herstellung von N-(3,5-Diacetamido-2,4,6-triiodbenzoyl)-, Alanin-, Sarkosin-, Leucin-, Serin-, N-Lactyl-lysin-, Asparaginsäure-, Glutaminsäuren, dadurch **ge-kennzeichnet,** daß man N-(3,5-Diacetamido-2,4,6-triiodbenzoyl)-,Glycin-, Alanin-, ß-Alanin-, Sarkosin-, Aminobuttersäure-, Leucin-, Serin-, N-Lactyl-lysin-, Asparaginsäure-, Glutaminsäureethylester in an sich bekannter Weise mit einer Lauge wie Natrium- oder Kaliumhydroxid bei Temperaturen von -5 bis +30 °C hydrolysieren läßt.

25. Verfahren zur Herstellung von N-(3,5-Diacetamido-2,4,6-triiodbenzoyl)-, Alanin-, Sarkosin-, Leucin-, Serin-, N-Lactyl-lysin-, Asparaginsäure-, Glutaminsäure-o- (oder p-)-Nitrophenylester, dadurch **gekennzeichnet,** daß man eine N-(3,5-Diacetamido-2,4,6-triiodbenzoyl)-, Alanin-, Sarkosin-, Leucin-, Serin-, N-Lactyl-lysin-, Asparaginsäure-, Glutaminsäure mit o- (oder p- )-Nitrophenol in N,N-Dimethylformamid/Tetrahydrofuran in Gegenwart von N,N-Dicyclohexylcarbodiimid, bei Temperaturen von 0 bis 30 °C reagieren läßt.

26. Verfahren zur Herstellung von N-[α-(O-Acetyl-lactyl)-ε-(3,5-diacetamido-2,4,6-triiodbenzoyl)]-lysinethylester (XXVII) und N-[ε(O-Acetyl-lactyl)-α-(3,5-diacetamido-2,4,6-triiodbenzoyl)]-lysinethylester (XXVIII), dadurch **gekennzeichnet,** daß man ε,N-Benzyloxycarbonyl-lysinethylester mit 3,5-Diacetamido-2,4,6-triiodbenzoylchlorid bzw. O-Acetyl-milchsäurechlorid in N,N-Dimethylformamid oder N,N-Dimethylacetamid in Gegenwart eines tertiären Amins wie Triethylamin oder Tributylamin, bei Temperaturen von 20 bis 60 °C reagieren läßt, die Schutzgruppe der Verbindungen XXIX und XX mit Bromwasserstoff in Essigsäure oder hydrogenolytisch (Wasserstoff-Palladium auf Aktivkohle) entfernt und anschließend α,N-(3,5-Diacetamido-2,4,6-triiodbenzoyl)-lysinethylester bzw. α,N-(O-Acetyl-lactyl)-lysinethylester mit O-Acetyl-milchsäurechlorid bzw. 3,5-Diacetamido-2,4,6-triiodbenzoylchlorid in N,N-Dimethylformamid oder N,N-Dimethylacetamid in Gegenwart eines tertiären Amins wie Triethylamin oder Tributylamin, bei Temperaturen von 20 bis 60 °C reagieren läßt.

27. Röntgenkontrastmittel für Vasographie, Urographie, Myelographie, Arthrographie, Fistulographie und Salpingographie, enthaltend nichtionische, wasserlösliche Derivate der 3,5-Diacetamido-2,4,6-triiodbenzoesäure nach einem der Ansprüche 1 bis 10.

## Claims

1. An N,N-[(3,5-diacetamido-2,4,6-triiodobenzoyl)aminoacyl]-N-methylglucamide of the general formula

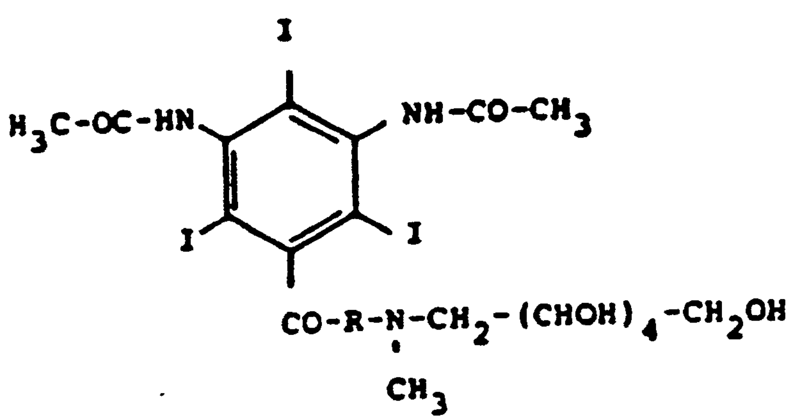

(I)

wherein R is an alanine, sarcosine, leucine, serine, N-lactyllysine, aspartic acid or glutamic acid residue.

2. N,N-[(3,5-Diacetamido-2,4,6-triiodobenzoyl)sarcosine]-N-methylglucamide (II).

3. N,N-[(3,5-Diacetamido-2,4,6-triiodobenzoyl)-L-alanine]-N-methylglucamide (III).

4. N,N-[(3,5-Diacetamido-2,4,6-triiodobenzyl)-L-leucine]-N-methylglucamide (IV).

5. N,N-[(3,5-Diacetamido-2,4,6-triiodobenzoyl)-L-serine]-N-methylglucamide (V).

6. N,N-[(3,5-Diacetamido-2,4,6-triiodobenzoyl)-L-aspartic acid]-bis-N-methylglucamide (VI).

7. N,N-[(3,5-Diacetamido-2,4,6-triiodobenzoyl)-DL-aspartic acid]-bis-N-methylglucamide (VII).

8. N,N-[(3,5-Diacetamido-2,4,6-triiodobenzoyl)-L-glutamic acid]-bis-N-methylglucamide (VIII).

9. N,N-[α-Lactyl-ε-(3,5-diacetamido-2,4,6-triiodobenzoyl)lysine]-N-methylglucamide (IX).

10. N,N-[ε-Lactyl-α-(3,5-diacetamido-2,4,6-triiodobenzoyl)lysine]-N-methylglucamide (X).

11. An N-(3,5-diacetamido-2,4,6-triiodobenzoyl)amino acid of general formula XI:

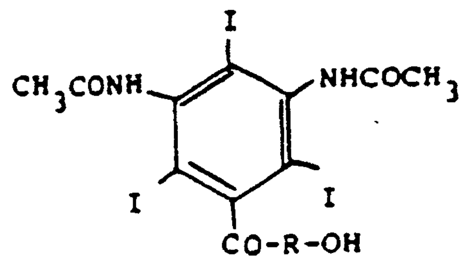

wherein R is an alanine, sarcosine, leucine, serine, N-lactyllysine, aspartic acid or glutamic acid residue, and an N-(3,5-diacetamido-2,4,6-triiodobenzoyl)amino acid ester of general formula XII:

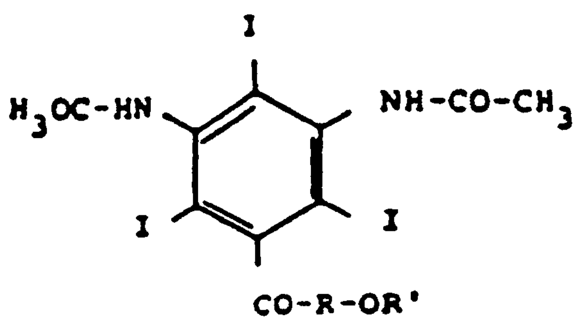

wherein R is an alanine, sarcosine, leucine, serine, N-lactyllysine, aspartic acid or glutamic acid residue and R' is an alkyl or o- or p-nitrophenyl ester.

12. N-(3,5-Diacetamido-2,4,6-triiodobenzoyl)sarcosine (XIII), its ethyl ester (XIV) and its o-nitrophenyl ester (XV).

13. N-(3,5-Diacetamido-2,4,6-triiodobenzoyl)-L-alanine (XVI), its ethyl ester (XVII) and its o-nitrophenyl ester (XVIII).

14. N-(3,5-Diacetamido-2,4,6-triiodobenzoyl)-L-leucine (XIX), its ethyl ester (XX) and its o-nitrophenyl ester (XXI).

15. N-(3,5-Diacetamido-2,4,6-triiodobenzoyl)-L-serine (XXII), its ethyl ester (XXIII) and its o-nitrophenyl ester

(XXIV).

**16.** N-(3,5-Diacetamido-2,4,6-triiodobenzoyl)-L-glutamic acid (XXV) and its diethyl ester (XXVI).

**17.** N-[α-(O-Acetyllactyl)-ε-(3,5-diacetamido-2,4,6-triiodobenzoyl)]-L-lysine ethyl ester (XXVII).

**18.** N-[ε-(O-Acetyllactyl)-α-(3,5-diacetamido-2,4,6-triiodobenzoyl))-L-lysine ethyl ester (XXVIII).

**19.** N-[ε-Benzyloxycarbonyl-α-(3,5-diacetamido-2,4,6-triiodobenzoyl)]-L-lysine ethyl ester (XXIX).

**20.** N-[ε-Benzyloxycarbonyl-α-(O-acteyllactyl)]-L-lysine ethyl ester (XXX) of the formula

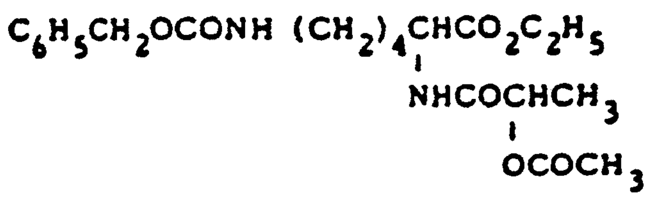

(XXX)

**21.** A method of preparing an N,N-[(3,5-diacetamido-2,4,6-triiodobenzoyl)aminoacyl]-N-methylglucamide of the formula

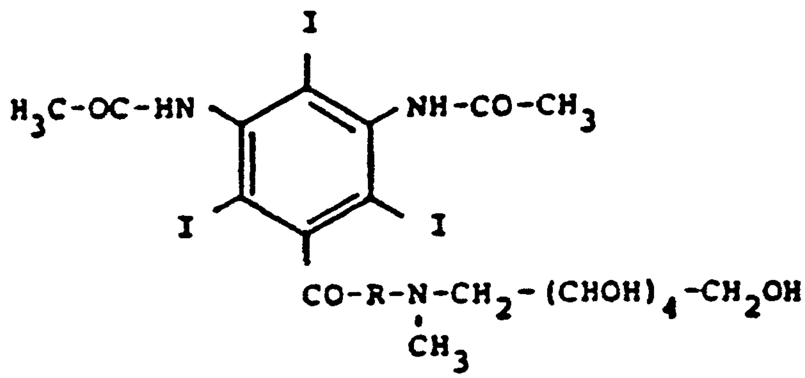

(I)

according to Claim 1, characterized in that an ester of the general formula

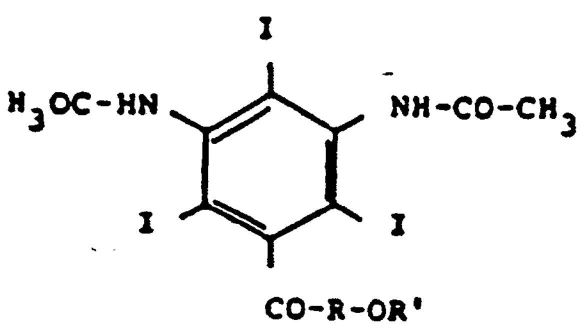

(XII)

wherein R is an alanine, sarcosine, leucine, serine, N-lactyllysine, aspartic acid or glutamic acid residue and R' is an alkyl or o- or p-nitrophenyl ester residue, is reacted with N-methylglucamine in a manner known per se, in a cation-solvating solvent such as N,H-dimethylformamide, N,N-dimethylacetamide or dimethyl sulphoxide, in the presence of an alkali metal carbonate, for example at temperatures of 20 to 80 °C.

**22.** A method of preparing compounds of general formula I, characterized in that acids of the general formula

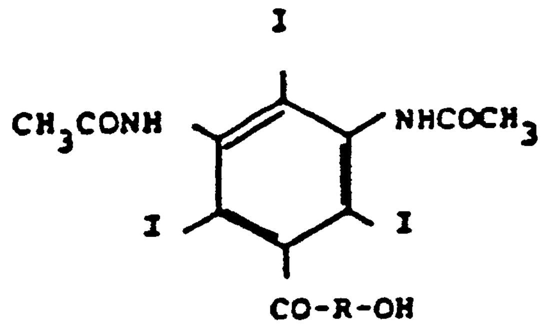

(XI)

wherein R is an alanine, sarcosine, leucine, serine, N-lactyllysine, aspartic acid or glutamic acid residue, is reacted with a lower alkyl ester of chloroformic acid in a cation-solvating solvent such as N,N-dimethylformamide or N,N-dimethylacetamide, in the presence of a tertiary amine such as triethylamine or 4-methylmorpholine, at temperatures of -15 to 0° C, and the anhydride is then reacted with N-methylglucamine in a solvent such as N,N-dimethylformamide, N,N-dimethylacetamide, methanol, ethanol or water, at temperatures of -10 to +35° C.

**23.** A method of preparing the ethyl ester of N-(3,5-diacetamido-2,4,6-triiodobenzoyl)-alanine, -sarcosine, -leucine, -serine, -N-lactyllysine, -aspartic acid or -glutamic acid, characterized in that 3,5-diacetamido-2,4,6-triiodobenzoyl chloride is reacted with the ethyl ester of glycine, alanine, β-alanine, sarcosine, aminobutyric acid, leucine, serine, N-lactyllysine, aspartic acid or glutamic acid in N,N-dimethylformamide or N,N-dimethylacetamide, in the presence of a tertiary amine such as triethylamine, tributylamine or pyridine, at temperatures of 20 to 60° C.

**24.** A method of preparing N-(3,5-diacetamido-2,4,6-triiodobenzoyl)-alanine, -sarcosine, -leucine, -serine, -N-lactyllysine, -aspartic acid or -glutamic acid, characterized in that the ethyl ester of N-(3,5-diacetamido-2,4,6-triiodobenzoyl)-glycine, -alanine, -β-alanine, -sarcosine, -aminobutyric acid, -leucine, -serine, -N-lactyllysine, -aspartic acid or -glutamic acid is hydrolyzed in a manner known per se, with a lye such as sodium or potassium hydroxide, at temperatures of -5 to +30° C.

**25.** A method of preparing the o- or p-nitrophenyl ester of N-(3,5-diacetamido-2,4,6-triiodobenzoyl)-alanine, -sarcosine, -leucine, -serine, -N-lactyllysine, -aspartic acid or -glutamic acid, characterized in that N-(3,5-diacetamido-2,4,6-triiodobenzoyl)-alanine, -sarcosine, -leucine, -serine, -N-lactyllysine, -aspartic acid or -glutamic acid is reacted with o- or p-nitrophenol in N,N-dimethylformamide/tetrahydrofuran, in the presence of N,N-dicyclohexylcarbodiimide, at temperatures of 0 to 30° C.

**26.** A method of preparing N-[α-(O-acetyllactyl)-ε-(3,5-diacetamido-2,4,6-triiodobenzoyl)]lysine ethyl ester (XXVII) and N-[ε-(O-acetyllactyl)-α-(3,5-diacetamido-2,4,6-triiodobenzoyl)]lysine ethyl ester (XXVIII), characterized in that ε,N-benzyloxycarbonyllysine ethyl ester is reacted with 3,5-diacetamido-2,4,6-triiodobenzoyl chloride or O-acetyllactic acid chloride in N,N-dimethylformamide or N,N-dimethylacetamide, in the presence of a tertiary amine such as triethylamine or tributylamine, at temperatures of 20 to 60° C, the protecting group of compounds XXIX and XXX is removed with hydrogen bromide in acetic acid or by hydrogenolysis (hydrogen/palladium on activated charcoal), and α,N-(3,5-diacetamido-2,4,6-triiodobenzoyl)lysine ethyl ester or α,N-(O-acetyllactyl)lysine ethyl ester is then reacted with O-acetyllactic acid chloride or 3,5-diacetamido-2,4,6-triiodobenzoyl chloride in N,N-dimethylformamide or N,N-dimethylacetamide, in the presence of a tertiary amine such as triethylamine or tributylamine, at temperatures of 20 to 60° C.

**27.** An X-ray contrast medium for vasography, urography, myelography, arthrography, fistulography and salpingography, containing a non-ionic water-soluble 3,5-diacetamido-2,4,6-triiodobenzoic acid derivative according to any one of Claims 1 to 10.

**Revendications**

1. N,N-[(3,5-diacétamido-2,4,6-triiodobenzoyl)-aminoacyl]-N-méthylglucamide de formule générale:

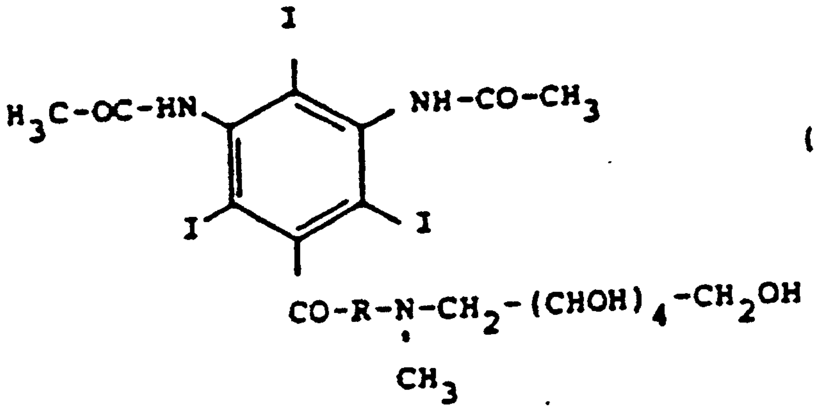

(I)

dans laquelle R est dérivé d'un radical alanine, sarcosine, leucine, sérine, N-lactyl-lysine, d'acide aspartique ou d'acide glutamique.

2. N,N-[(3,5-diacétamido-2,4,6-triiodobenzoyl)-sarcosine]-N-méthylglucamide (II).

3. N,N-[(3,5-diacétamido-2,4,6-triiodobenzoyl)-L-alanine]-N-méthylglucamide (III).

4. N,N-[(3,5-diacétamido-2,4,6-triiodobenzoyl)-L-leucine]-N-méthylglucamide (IV).

5. N,N-[(3,5-diacétamido-2,4,6-triiodobenzoyl)-L-serine]-N-méthylglucamide (V).

6. N,N-[(3,5-diacétamido-2,4,6-triiodobenzoyl)-L-acide aspartique]-bis-méthylglucamide (VI).

7. N,N-[(3,5-diacétamido-2,4,6-triiodobenzoyl)-DL-acide aspartique]-bis-N-méthylglucamide (VII).

8. N,N-[(3,5-diacétamido-2,4,6-triiodobenzoyl)-L-acide glutamique]-bis-N-méthylglucamide (VIII).

9. N,N-[α-lactyl-ε-(3,5-diacétamido-2,4,6-triiodobenzoyl)-lysine]-N-méthylglucamide (IX).

10. N,N-[ε-lactyl-α-(3,5-diacétamido-2,4,6-triiodobenzoyl)-lysine]-N-méthylglucamide (X).

11. Un acide aminé N-(3,5-diacétamido-2,4,6-triiodobenzoyl) de formule générale XI:

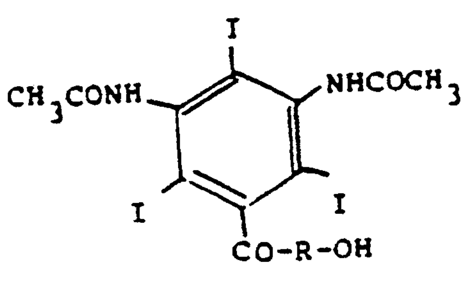

dans laquelle R est un radical dérivé d'alanine, sarcosine, leucine, sérine, N-lactyl-lysine, d'acide aspartique, d'acide glutamique, et les esters de l'acide aminé (3,5-diacétamido-2,4,6-triiodobenzoyl) de formule générale XII:

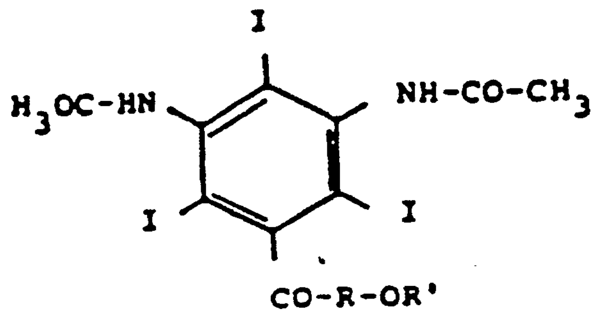

dans laquelle R est un radical dérivé d'alanine, sarcosine, leucine, sérine, N-lactyl-lysine, d'acide aspartique, d'acide glutamique et R' est un radical alkyle ou o- ou p-nitrophénylester.

**12.** N-[(3,5-diacétamido-2,4,6-triiodobenzoyl)-sarcosine (XIII), son ester éthylique (XIV), et son ester o-nitrophénylique (XV).

**13.** N-(3,5-diacétamido-2,4,6-triiodobenzoyl)-L-alanine (XVI), son ester éthylique (XVII), et son ester o-nitrophénylique (XVIII).

**14.** N-(3,5-diacétamido-2,4,6-triiodobenzoyl)-L-leucine (XIX), son ester éthylique (XX), et son ester o-nitrophénylique (XXI).

**15.** N-(3,5-diacétamido-2,4,6-triiodobenzoyl)-L-sérine (XXII), son ester éthylique (XXIII), et son ester o-nitrophénylique (XXIV).

**16.** N-(3,5-diacétamido-2,4,6-triiodobenzoyl)-L-acide glutamique (XXV), et son diester éthylique (XXVI).

**17.** Ester éthylique de la N-[α-(O-acétyl-lactyl)-ε-(3,5-diacétamido-2,4,6-triiodobenzoyl)]-L-lysine (XXVII).

**18.** Ester éthylique de la N-[ε-(O-acétyl-lactyl)-α-(3,5-diacétamido-2,4,6-triiodobenzoyl)]-L-lysine (XXVIII).

**19.** Ester éthylique de la N-[ε-benzyloxycarbonyl-α-(3,5-diacétamido-2,4,6-triiodobenzoyl)]-L-lysine (XXIX).

**20.** Ester éthylique de la N-[ε-benzyloxycarbonyl-α-(O-acétyl-lactyl)]-L-lysine (XXX):

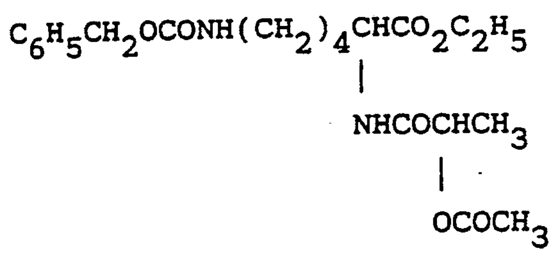

(XX)

**21.** Un procédé de préparation de N,N-[(3,5-diacétamido-2,4,6-triiodobenzoyl)-aminoacyl]-N-méthylglucamide de formule:

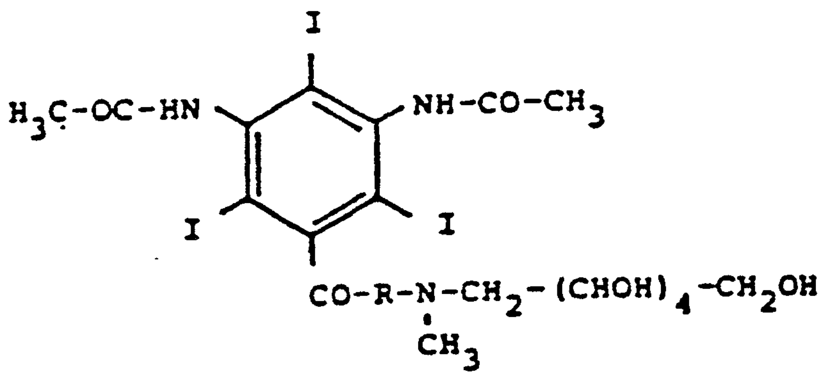

(I)

selon la revendication 1, caractérisé en ce qu'on fait réagir un ester de formule générale:

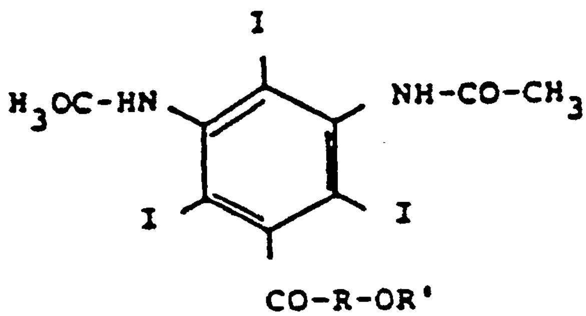

dans laquelle R est un radical dérivé d'alanine, sarcosine, leucine, sérine, N-lactyl-lysine, d'acide aspartique, d'acide glutamique et R' est un radical alkyle ou o- ou p-nitrophényle, selon une méthode classique avec de la N-méthylglucamine dans un milieu de solvatation des cations, tels que la N,N-diméthylformamide, la N,N-diméthylacétamide ou le diméthylsulfoxyde en présence d'un carbonate alcalin, à une température de 20 à 80 ° C.

22. Procédé de préparation d'un composé de formule générale I, caractérisé en ce qu'on transforme un acide de formule générale:

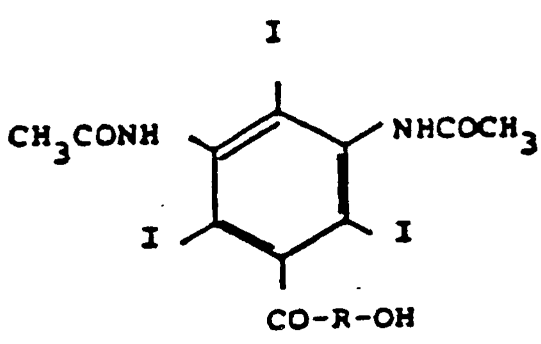

(XI)

dans laquelle R est un radical dérivé d'alanine, sarcosine, leucine, sérine, N-lactyl-lysine, d'acide aspartique, d'acide glutamique, avec un ester d'alkyle inférieur de l'acide chloroformique dans un milieu de solvatation des solvants tel que la N,N-diméthylformamide ou la N,N-diméthylacétamide en présence d'une amine tertiaire telle que la triéthylamine ou la 4-méthylmorpholine à une température de -15 à 0 ° C et qu'ensuite on fait réagir l'anhydride résultant avec de la N-méthylglucamine dans un solvant tel que la N,N-diméthylformamide, la N,N-diméthylacétamide, le méthanol, l'éthanol ou de l'eau à des températures de -10 à + 35 ° C.

**23.** Procédé de préparation d'ester éthylique de N-(3,5-diacétamido-2,4,6-triiodobenzoyl)-, alanine, sarcosine, leucine, sérine, N-lactyl-lysine, d'acide aspartique, d'acide glutamique, caractérisé en ce qu'on fait réagir du chlorure de 3,5-diacétamido-2,4,6-triiodobenzoyle avec un ester éthylique de glycine, alanine, $\beta$-alanine, sarcosine, acide aminobutyrique, leucine, sérine, N-lactyl-lysine, d'acide aspartique, d'acide glutamique dans de la N,N-diméthylformamide ou de la N,N-diméthylacétamide en présence d'une amine tertiaire telle que la triéthylamine, la tributylamine ou la pyridine à une température de 20 à 60 °C.

**24.** Procédé de préparation de N-(3,5-diacétamido-2,4,6-triiodobensoyl)-, alanine, sarcosine, leucine, sérine, N-lactyl-lysine, d'acide aspartique, d'acide glutamique, caractérisé en ce qu'on hydrolyse un ester éthylique de N-(3,5-diacétamido-2,4,6-triiodobenzoyl)-, glycine, alanine, $\beta$-alanine, sarcosine, acide amino-butyrique, leucine, sérine, N-lactyl-lysine, d'acide aspartique, d'acide glutamique selon des procédés connus avec une base telle que de l'hydroxyde de sodium ou de potassium à une température de -5 à +30 °C.

**25.** Procédé de préparation d'ester o- ou p-nitrophénylique de N-(3,5-diacétamido-2,4,6-triiodobenzoyl)-, alanine, sarcosine, leucine, sérine, N-lactyl-lysine, d'acide aspartique, d'acide glutamique, caractérisé en ce qu'on fait réagir de la N-(3,5,-diacétamido-2,4,6-triiodobenzoyl)-, alanine, sarcosine, leucine, sérine, N-lactyl-lysine, d'acide aspartique, d'acide glutamique avec du o- (ou p-)-nitrophénol dans du N,N-diméthylformamide/tétrahydrofurane en présence de N,N-dicyclohexylcarbodiimide, à une température de 0 à 30 °C.

**26.** Procédé de préparation d'ester éthylique de N-[$\alpha$-(O-acétyl-lactyl)-$\epsilon$-(3,5-diacétamido-2,4,6-triiodobenzoyl)]-lysine (XXVII) et d'ester éthylique de N-[$\epsilon$(O-acétyl-lactyl)-$\alpha$-(3,5-diacétamido-2,4,6-triiodobenzoyl)]-lysine (XXVIII), caractérisé en ce qu'on fait réagir l'ester éthylique de $\epsilon$,N-benzyloxycarbonyl-lysine avec du chlorure de 3-5-diacétamido-2,4,6-triiodobenzoyle et du chlorure d'acide O-acétyle lactique respectivement dans de la N,N-diméthylformamide ou de la N,N-diméthyl-acétamide en présence d'une amine tertiaire telle que la triéthylamine ou la tributylamine à une température de 20 à 60 °C, en ce qu'on enlève le groupe protecteur des composés XXIX et XXX avec du bromure d'hydrogène dans de l'acide acétique ou de manière hydrogénante (hydrogène/palladium sur charbon activé) et que subséquemment on fait réagir l'ester éthylique $\alpha$,N-(3,5-diacétamido-2,4,6-triiodobenzoyl)-lysine et l'ester éthylique $\alpha$,N-O-(acétyl-lactyl)-lysine respectivement avec du chlorure d'acide O-acétyle lactique et du chlorure de 3,5-diacétamido-2,4,6-triiodobenzoyle respectivement dans de la N,N-diméthylformamide ou de la N,N-diméthylacétamide en présence d'une amine tertiaire, la triéthylamine ou la tributylamine à une température de 20 à 60 °C.

**27.** Traceur radiographique pour la vasographie, l'urographie, la myélographie, l'arthrographie, la fistulographie et la salpingographie, contenant un dérivé non ionique soluble dans l'eau de l'acide 3,5-diacétamido-2,4,6-triiodobenzoïque selon l'une quelconque des revendications 1 à 10.